# EUROPEAN PATENT APPLICATION

(11) **EP 3 943 074 A1**
(43) Date of publication of application: **26.01.2022**
(21) Application number: 20187533.3
(22) Date of filing: 23.07.2020
(51) Int. Cl.: A61K 9/20, A61K 31/7048, A61P 11/06, A61P 11/08

(54) **ORAL UNIT DOSAGE FORM OF IVERMECTIN**

(71) Applicant: Chemo Research SL, 28050 Madrid (ES)
(72) Inventor: LANUSSE, Carlos, Tandil, Buenos Aires, 7000 (AR); KROLEWIECKI, Alejandro, Ciudad Autónoma de Buenos Aires, 1426 (AR)
(74) Representative: Schön, Christoph

(57) **Abstract**

The present invention discloses an oral solid unit dosage form comprising an amount of 3 to 25 mg ivermectin in at least one pharmaceutically acceptable excipient, and a method of treating subjects by administering an oral solid unit dosage form comprising an amount of 3 to 25 mg ivermectin in at least one pharmaceutically acceptable carrier.

## Description

### Field of the invention

The present invention relates to the field of oral solid unit dosage forms comprising ivermectin, more preferably to oral dosage unit forms comprising an amount of 3 to 25 mg ivermectin in at least one pharmaceutically acceptable excipient, and a method of treating subjects by administering an oral solid unit dosage form comprising an amount of 3 to 25 mg ivermectin in at least one pharmaceutically acceptable carrier.

### Background of the invention

Ivermectin is a member of the avermectin class, which has been shown in immunopharmacological studies to exert anti-inflammatory effects by inhibiting lipopolysaccharide-induced production of inflammatory cytokines, such as tumor necrosis factor alpha and interleukin(IL)-1β, while upregulating the anti-inflammatory cytokine IL-10. It is a semi-synthetic derivative isolated from the fermentation of *Streptomyces avermitilis,* that belongs to the avermectin family of macrocyclic lactones. Ivermectin is a mixture containing 5-O-demethyl-22,23-dihydroavermectin Ala plus 5-O-demethyl-25-de(1-methylpropyl)-25-(1-methylethyl)-22,23-dihydroavermectin Ala, generally referred to as 22,23-dihydroavermectin B1a and B1b or H2B1a and H2B1b, respectively. The respective empirical formulas of H2B1a and H2B1b are C₄₈H₇₄O₁₄ and C₄₇H₇₂O₁₄ with molecular weights of 875.10 and 861.07, respectively.

Ivermectin is a macrocyclic lactone derivative, its therapeutic effect is thought to be prominently due to its anti-inflammatory properties, similar to that of other macrolides. Avermectin has been reported to exert anti-inflammatory effects by inhibiting lipopolysaccharide-induced production of inflammatory cytokines. In addition to its anti-inflammatory mode of action, ivermectin possesses antiparasitic properties. Its predecessor, avermectin, is an antiparasitic agent of agricultural importance first isolated in 1974. Several studies support ivermectin's role in the effective oral treatment of cutaneous demodicidosis (in combination with topical permethrin cream) and scabies, as well as topical treatment of head lice. Ivermectin causes death of parasites, primarily through binding selectively and with high affinity to glutamate-gated chloride channels, which occur in invertebrate nerve and muscle cells. This leads to the interruption of nerve impulses, causing paralysis and death of parasitic organisms. Ivermectin is known to act on Demodex mites in localized and generalized demodicidosis in animals and in humans.

At present in the US, Brazil and France oral tablets comprising 3 mg of ivermectin (see for example Stromectol^{®}, NDA 050742, Merck Sharpe and Dohme) are approved for the treatment of Onchoceriasis and Strongyloidiasis of the intestinal tract.

Furthermore, ivermectin has previously been studied as a therapeutic option for viral infections with in vitro data showing some activity against a broad range of viruses, including HIV, Dengue, Influenza and Zika virus.

The world is currently facing SARS CoV 2 (severe acute respiratory syndrome coronavirus 2, the cause of COVID-19), for which highly effective therapies still are to be developed. So far several established drugs including antiviral agents, antibiotics and anti-inflammatory agents have been administered as off-label therapies. These drugs have mostly been given without controls and, therefore, valid evidence of efficacy to be demonstrated by e.g. randomized controlled trials (RCTs) are missing so far.

Recently Caly et al. ("The FDA-approved drug ivermectin inhibits the replication of SARS-CoV-2 in vitro", Antiviral Research 178 (2020), pages 1 to 4) report that ivermectin may also inhibit the replication of SARS-CoV-2 virus *in vitro* when using ivermectin at 5 µM.

However, the concentration resulting in 50% inhibition (IC50: 2 µM) was >35-times higher than the maximum plasma concentration (Cₘₐₓ) of 0.05 µM after oral administration of the approved dose of 200 µg/kg.

### Summary of the invention

Based on this state of the art, it is an object of the present invention to provide oral solid unit dosage forms comprising ivermectin which may be used in treating subjects suffering from a coronavirus-associated disease, such as COVID-19.

Furthermore, another object of the present invention is to provide a method of treating subjects suffering from a coronavirus-associated disease, such as COVID-19.

In addition, it is an object of the present invention to provide an oral solid unit dosage form comprising ivermectin for use in the treatment of subjects suffering from COVID-19, especially to provide an oral solid unit dosage form for use in such a treatment which provides a high safety margin and is at the same time efficient in reducing or alleviating symptoms of a coronavirus-associated disease such as a lower mortality or a shortly period of hospitalization, and/or reducing viral load and/or in reducing the viral RNA within days.

In order to solve the above object, the present invention provides an oral solid unit dosage form comprising an amount of 3 to 25 mg ivermectin or a pharmaceutically acceptable salt or solvate thereof in at least one pharmaceutically acceptable excipient.

Furthermore, the present invention provides a method of treating subjects suffering from a coronavirus-associated disease, preferably COVID-19 by administering an oral solid unit dosage form comprising an amount of 3 to 25 mg ivermectin or a pharmaceutically acceptable salt or solvate thereof in at least one pharmaceutically acceptable carrier.

In addition, the present invention provides an oral solid unit dosage form comprising ivermectin or a pharmaceutically acceptable salt or solvate thereof in at least one pharmaceutically acceptable carrier for use in the treatment of subjects suffering from a coronavirus-associated disease, preferably COVID-19.

### Description of the Drawings

Figure 1 illustrates a percentage of reduction of viral load in patients affected by COVID-19 after administration of ivermectin tablets according to the present invention;
Figure 2A shows a correlation between ivermectin (IVM) concentration and percentage of reduction in viral load in the treated group; and
Figure 2B shows a correlation between ivermectin (IVM) concentration and Rate Decay (day⁻¹) (0.54 day⁻¹ Decay rate for the patient with 183 ng/ml IVM concentration).

### Detailed description of the invention

As used herein, the term "subject" means any animal, preferably a mammal, most preferably a human, to whom will be or has been administered compounds or unit dosage forms according to embodiments of the invention. Preferably, a subject is in need of, or has been the object of observation or treatment or prevention of a coronavirus-associated disease, preferably COVID-19.

In one embodiment, "treatment" or "treating" refers to an amelioration, prophylaxis, or reversal of a disease or disorder, or of at least one discernible symptom thereof. In another embodiment, "treatment" or "treating" refers to an amelioration, prophylaxis, or reversal of at least one measurable physical parameter related to the disease or disorder being treated, not necessarily discernible in or by the mammal. In yet another embodiment, "treatment" or "treating" refers to inhibiting or slowing the progression of a disease or disorder, either physically, e.g., stabilization of a discernible symptom, physiologically, e.g., stabilization of a physical parameter, or both. In yet another embodiment, "treatment" or "treating" refers to delaying the onset of a disease or disorder.

The term "viral load" is well known to a person skilled in the art. The term "viral load" is a measure of the severity of an active viral infection, and can be determined by methods known to the person skilled in the art. As an Example, the viral load can be calculated by estimating the live amount of virus in an involved body fluid such as a number of RNA copies *per* milliliter of blood plasma. For example, the viral load can be determined by a real-time (RT) PCR test.

According to a first aspect the present invention provides an oral solid unit dosage form comprising an amount of 3 to 25 mg ivermectin or a pharmaceutically acceptable salt or solvate thereof in at least one pharmaceutically acceptable carrier.

In a preferred embodiment the oral solid unit dosage form comprises an amount 5 to 21 mg ivermectin or a pharmaceutically acceptable salt or solvate thereof, more preferably 7 to 20 mg, more preferably 8 to 19 mg, even more preferably 9 to 18 mg, most preferably 9 or 18 mg in at least one pharmaceutically acceptable carrier.

Typically, the ivermectin or a pharmaceutically acceptable salt or solvate thereof can be present in the composition of the oral solid unit dosage form according to the present invention in an amount of about 0.5% to about 70%, for example about 1% to about 60%, or about 1% to about 40%, or about 1% to 30% by weight relative to the total weight of the oral solid unit dosage form.

In a preferred embodiment of the first aspect, the oral solid unit dosage form is an immediate-release unit dosage form.

The solubility characteristics of the active ingredient ivermectin or a pharmaceutically acceptable salt or solvate thereof in immediate-release unit dosage forms are in accordance with the USP specification for immediate release tablets containing ivermectin or a pharmaceutically acceptable salt or solvate thereof as the active ingredient. For example, in the case of ivermectin containing immediate-release tablets, more than 75%, preferably more than 80%, more preferably more than 90% of the ivermectin contained in the unit dosage form can be detected in the dissolution medium within 45 minutes during agitation under appropriate conditions when using an USP device 2 (paddle) at 50 rpm in 0.01M phosphate buffer, pH 7 with 0.5% of sodium dodecyl sulfate (see also USP43-NF38).

With respect to the active ingredient, i.e., ivermectin, present in said unit dosage form, it is well known in the respective field of the art that ivermectin may be used as amorphous ivermectin or crystalline ivermectin.

According to the present invention it was found that in the oral solid unit dosage form, especially in an immediate-release unit dosage form preferably crystalline ivermectin particles should be present.

Said crystalline ivermectin particles for example may be produced by dissolving crude ivermectin in ethanol and adding formamide to the ethanol solution. Subsequently water should be added over said solution and the resulting solution should be cooled for precipitation.

After collecting the crystalline ivermectin particles, the wet crystalline ivermectin particles may be further slurried in a mixture of ethanol and water, isolated by filtration or centrifugation, dried and sieved so as to produce crystalline ivermectin particles.

According to the present invention it is preferred to use crystalline ivermectin particles having a particle size distribution D(v, 0.5) of 5 to 40 µm, more preferable of 10 to 25 µm and/or a particle size distribution D(v, 0.9) of 50 to 90 µm, more preferable of 55 to 75 µm.

With respect to the oral solid unit dosage form, said unit dosage form usually is produced by a process comprising a step of dry blending the active ingredient and at least one pharmaceutically acceptable excipient, followed by a milling or sieving step, to prepare a substantially finely dispersed, uniform powder blend, which can then be further processed into the finished unit dosage form such as tablets, capsules, and powders.

The solid oral unit dosage forms according to the present invention are usually prepared by a process which comprises at least one dry blending and milling or sieving process step, preferably a first mixing step, wherein part of the pharmaceutically acceptable excipients and the active ingredient are mixed, a milling or sieving step in order to form a uniform powder mixture, a further mixing step, wherein the remaining pharmaceutically acceptable excipients are mixed with the uniform powder mixture obtained in the sieving or milling step, and a step of compacting the resultant powder mixture to obtain a finished oral solid unit dosage form.

The dry blending and milling steps are carried out in order to prepare a powder blend having a substantially finely dispersed, uniform distribution of the active ingredient ivermectin within the at least one pharmaceutically acceptable excipient base wherein the final blend is comprised of primary and tightly bound secondary particles. The creation of a final blend comprised of primary and secondary particles is a significant advantage of the process for producing an oral solid unit dosage form according to the present invention compared to a granulation process which is often used in order to produce oral solid unit dosage forms.

According to the present invention ivermectin particles and the at least one pharmaceutically acceptable excipient are co-milled so as to produce ivermectin particles having a particle size distribution D(v, 0.5) of 30 to 125 µm, more preferable of 45 to 110 µm and/or a particle size distribution D(v, 0.9) of 170 to 250 µm, more preferable of 190 to 210 µm in the final unit dosage form.

When the particle dimensions in the unit dosage form are more uniform, better flow of the active ingredient is achieved during encapsulation, sachet filling, or tableting, which results in a more substantially uniform finished unit dosage form.

Examples of the at least one pharmaceutical excipient that can be used include, but are not limited to:
(i) binders/fillers such as starch, microcrystalline cellulose, lactose, and dicalcium phosphate;
(ii) disintegrants such as starch, croscarmellose sodium, sodium starch glycolate, and cross-linked polyvinyl pyrrolidone;
(iii) glidants such as silica and talc;
(iv) lubricants such as stearic acid, magnesium stearate, and sodium stearyl fumarate;
(v) chelating agents such as ethylene diamine tetraacetic acid (EDTA), acetate or anhydrous citric acid;
(vi) colorants such as FD&C lake pigments;
(vii) flavoring agents such as natural and artificial flavors;
(viii) preservatives such as benzoic acid;
(ix) antioxidants such as butylated hydroxyanisole, and butylated hydroxytolulene; and
(x) pH modifiers such as citric acid and fumaric acid.

The process for producing an oral solid unit dosage form comprising ivermectin particles according to the present invention usually comprises a first dry blending step, followed by a milling step, which is in turn is optionally followed by a second blending step.

The first blending step may be used to ensure that the active ingredient and the at least one pharmaceutically acceptable excipient in the powder mixture are sufficiently distributed.

The last step, an optional step, usually is a second blending step. In this second blending step, a lubricant may be added and blended to the milled ivermectin-excipient mixture.

The above process- with or without the optional second blending step- results in a fine dispersion of the ingredients exhibiting good blend uniformity resulting in a finished unit dosage form exhibiting acceptable content uniformity and facilitates a more rapid dissolution of the prepared finished unit dosage form.

According to the present invention an oral solid unit dosage form containing as much as 3 to 25 mg of ivermectin or a pharmaceutically acceptable salt or solvate thereof, preferably 5 to 21 mg ivermectin or a pharmaceutically acceptable salt or solvate thereof, more preferably 7 to 20 mg, more preferably 8 to 19 mg, even more preferably 9 to 18 mg, most preferably 9 or 18 mg is contemplated by the invention.

Although any pharmaceutically acceptable excipient can be used in the present invention by itself or in combination with other pharmaceutically acceptable excipients, a preferred form of the invention contains at least one pharmaceutically acceptable excipient that promotes rapid water uptake, including but not limited to microcrystalline cellulose or starch. An even more preferred unit dosage form according to the present invention contains at least one pharmaceutically acceptable excipient that promotes rapid water uptake, including but not limited to microcrystalline cellulose or starch, and does not contain an excipient that deforms by brittle fracture. A non-limiting example of an excipient that deforms by brittle fracture is dicalcium phosphate. A yet even more preferred embodiment contains at least one pharmaceutically acceptable excipient that deforms plasticly and promotes rapid water uptake, including but not limited to microcrystalline cellulose or partially pregelatinized starch, and does not contain an excipient that deforms by brittle fracture.

According to the present invention usually the final blend of ivermectin and at least one pharmaceutically acceptable excipient is subjected to a compression step so as to form an oral solid unit dosage form.

Suitable further pharmaceutically acceptable excipients include fillers, binders, disintegrants, lubricants, and the like, as is known in the art. In embodiments in which the unit dosage form is produced by compression and also provides modified release of the active ingredient contained therein, the unit dosage form preferably further comprises a release modifying compressible excipient.

Fillers suitable for use in making unit dosage forms by compression methods include sugars including dextrose, sucrose, maltose and lactose; water-soluble compressible carbohydrates such as starch hydrolysates including sugar-alcohols including mannitol, sorbitol, maltitol, xylitol, and dextrin and maltodextrin (e.g., microcrystalline cellulose or other cellulose derivatives), water insoluble, brittle fracture materials (e.g., dicalcium phosphate, tricalcium phosphate, etc.), and mixtures thereof.

Typically, fillers may be presents in an amount from about 5% to about 98% by weight, preferably from 10% to 95% by weight, and more preferably from 15% to 90% by weight based on the total weight of the oral solid unit dosage form.

Suitable binders for preparing unit dosage forms by compression methods include anhydrous binders (e.g., polyvinylpyrrolidone, hydroxypropylmethylcellulose, and the like); wetting agents (such as acacia, alginate, agar, guar gum, low-cost soybeans, carrageenan, carboxymethyl cellulose, tara, gum arabic, tragacanth, pectin, xanthan, gellan, gelatin, maltodextrin, galactomannan, water-soluble compounds including fumaric acid, derivatives and mixtures thereof.

Typically, binders may be presents in an amount from about 0.5% to about 30% by weight, preferably from 1% to 25% by weight, and more preferably from 3% to 20% by weight based on the total weight of the oral solid unit dosage form.

Suitable disintegrants for preparing unit dosage forms by compression methods include sodium starch glycolate, crosslinked polyvinylpyrrolidone, crosslinked carboxymethylcellulose, starch and microcrystalline cellulose and the like.

Typically, disintegrating agents may be present in an amount from about 2% to about 50% by weight, preferably from about 5% to about 45% by weight, and more preferably from 10% to 40% by weight based on the total weight of the oral solid unit dosage form.

Suitable lubricants for preparing unit dosage forms by compression methods include long chain fatty acids and salts thereof such as magnesium stearate and stearic acid, talc, glycerides and waxes.

The lubricant may be present in an amount from about 0% to 5% by weight, preferably from about 0% to about 3% by weight based on the total weight of the oral solid unit dosage form.

Suitable glidants for producing unit dosage forms by compression methods include colloidal silicon dioxide and the like.

Pharmaceutically acceptable adjuvants suitable for preparing unit dosage forms by compression methods usually include preservatives, high-concentration sweeteners (such as aspartame, acesulfame potassium, scallalose and saccharin); flavoring agents, coloring agents, antioxidants, surfactants and wetting agents, and the like, and mixtures thereof.

For embodiments in which the unit dosage form is produced by compression, dry blending (i.e., direct compression) processes as known in the art may be used.

In dry blending (direct compression), the active ingredient or ingredients are mixed in a suitable mixer, rather than transferred directly to a press for compacting with the excipient. The final dry mixing method is then suitable for compression.

When the unit dosage form should be a tablet, rotary compressors known in the art can be used. In a rotary compressor, the powder of the metered volume is charged into the die cavity, which is followed by a densified position where the powder is densified between the upper and lower punches.

In some embodiments the oral solid unit dosage forms may be coated with a coating agent, such as ethylcellulose, methyl hydroxyethyl cellulose, povidone, gelatin, hydroxypropyl cellulose, hypromellose, cellulose acetate phthalate, an acrylate polymer or hydroxymethyl propyl cellulose.

In some embodiments when tablets are used as the solid oral unit dosage forms, said tablets are produced in the form of scored tablets. Such scored tablets comprising at least ivermectin as the active ingredient, provide a benefit in that the dosage amount to be administered can be easily achieved so that a patient will always take the minimum dose to be administered.

In a second aspect the present invention provides a method of treating subjects suffering from a coronavirus-associated disease, preferably from COVID-19 by administering an oral solid unit dosage form comprising an amount of 3 to 25 mg ivermectin or a pharmaceutically acceptable salt or solvate thereof, preferably 5 to 21 mg ivermectin or a pharmaceutically acceptable salt or solvate thereof, more preferably 7 to 20 mg, more preferably 8 to 19 mg, even more preferably 9 to 18 mg, in at least one pharmaceutically acceptable carrier.

In previous studies in 1995 and 1999 for *Onchocerca volvulus* in onchoceriasis patients (see Awadzi, Opoku et al. 1995; Awadzi, Attah et al. 1999) it was shown in a double-blind placebo-controlled trial that increasing doses of ivermectin from 150 µg/kg/day to 1600 µg/kg/day were well tolerated by the patients.

Therefore, in view of the findings by Caly et al. mentioned above, that the replication of SARS-CoV-2 virus in vitro could be inhibited when administering ivermectin at 5 µM, it could be expected that high amounts of ivermectin should be most promising in treating patients suffering from COVID-19. In a second *in-vitro* test the investigators found that the estimated half maximal inhibitory concentration (IC50) of ivermectin amounts to 2µM.

However, such high doses used in the *in vitro* tests should not be feasible when treating living subjects suffering from coronavirus-associated diseases in view of side effects often seen in patients suffering for example from COVID-19 such as negative impacts on the liver and/or kidney function.

Furthermore, hyperinflammation and cytokine storm syndromes leading to serve acute respiratory distress syndrome, vascular damage and high risk of multiorgan failure have been described in COVID-19 patients. Highly increased release of tumor-necrosis factor alpha, interleukin-6, interleukin-7, interleukin-2 and macrophage inflammatory protein 1-alpha seem to play a key role in the COVID-19-induced cytokine storm syndrome/hyperinflammation. Such increased release of interleukin-6 can be found in several known hyperinflammatory/cytokine storm syndrome such as idiopathic multicentric Castleman disease, secondary hemophagocytic lymphohistiocytosis driven by viral infections or malignancies or CAR-T cell therapy related cytokine release syndromes leading to endothelial disfunctions and disruption of the blood-brain-barrier.

In addition, Buzhdygan et al. provided further evidence for a direct impact of the viral S1 protein on the barrier integrity of the human blood-brain-barrier in an advanced 3D microfluid model. Such impairment of the integrity of the blood-brain-barrier in COVID-19 patients might induce a pathological status which allows ivermectin, which under physiological conditions cannot pass the blood-brain barrier due to the mdr-1 gene encoded p-glycoprotein, to be transferred through the blood-brain barrier. In such pathological condition the high safety margin currently assumed for ivermectin would be jeopardized and serve neurological disfunctions may occur (see also the recently published studies of Chandler et al. ["Serious neurological adverse Events after Ivermectin- Do they occur beyond the indication of Onchocerciasis?"] which indicate a correlation of secondary impairment of the blood-brain-barrier and the occurrence of neurological adverse effects in patients receiving ivermectin).

In contrast, according to the present invention it was found that in order to treat subjects suffering from a coronavirus-associated disease, preferably COVID-19, the above described oral solid unit dosage forms comprising an amount of 3 to 25 mg of ivermectin, preferably 5 to 21 mg ivermectin or a pharmaceutically acceptable salt or solvate thereof, more preferably 7 to 20 mg, more preferably 8 to 19 mg, even more preferably 9 to 18 mg, should be administered in such a way that the patients are treated with 400 to 1200 µg/kg/day ivermectin, more preferably with 450 to 900 µg/kg/day ivermectin, even more preferably with 500 to 800 µg/kg/day ivermectin, even more preferably 480 to 800 µg/kg/day ivermectin or a pharmaceutically acceptable salt or solvate thereof over a period of one to seven days, preferably two to five days, more preferably two to three days. This finding is clearly unexpected and surprising because these doses are much higher than a dose of 200 µg/kg/day ivermectin currently approved by the FDA and at the same time provide an excellent result in lowering COVID-19 related symptoms without significantly increasing the number of adverse events.

According to the present invention it has been surprisingly found that by administering an amount of with 400 to 1200 µg/kg/day ivermectin, more preferably with 450 to 900 µg/kg/day ivermectin, even more preferably with 500 to 800 µg/kg/day ivermectin, even more preferably 480 to 800 µg/kg/day ivermectin or a pharmaceutically acceptable salt or solvate thereof over a period of one to seven daysover a period of one to seven days, preferably one to five days patients suffering from COVID-19 resulted in a significant reduction of viral load or viral RNA after end of administration cycle.

In addition, in a third aspect the present invention provides an oral solid unit dosage form comprising ivermectin in at least one pharmaceutically acceptable carrier for use in the treatment of subjects suffering from coronavirus associated diseases in humans, including COVID-19.

In a preferred embodiment the oral solid unit dosage form for use in the treatment of subjects suffering from a coronavirus associated disease the active ingredient ivermectin may be present in an amount of from 3 to 25 mg, preferably 5 to 21 mg ivermectin or a pharmaceutically acceptable salt or solvate thereof, more preferably 7 to 20 mg, more preferably 8 to 19 mg, even more preferably 9 to 18 mg.

In said preferred embodiment of an oral solid unit dosage form comprising ivermectin in an amount of 3 to 25 mg, preferably 5 to 21 mg ivermectin or a pharmaceutically acceptable salt or solvate thereof, more preferably 7 to 20 mg, more preferably 8 to 19 mg, even more preferably 9 to 18 mg, in at least one pharmaceutically acceptable carrier for use in the treatment of subjects suffering from a coronavirus-associated disease, preferably COVID-19, said oral solid dosage form is administered in an amount of with 400 to 1200 µg/kg/day ivermectin, more preferably with 450 to 900 µg/kg/day ivermectin, even more preferably with 500 to 800 µg/kg/day ivermectin, even more preferably 480 to 800 µg/kg/day ivermectin or a pharmaceutically acceptable salt or solvate thereof over a period of one to seven days over a period of one to seven days, preferably one to five days patients suffering from COVID-19, resulting in an improvement in comparison with patients of a non-treated control group or standard care control group. Efficacy parameters are selected from the group consisting of a reduction of the disease symptoms and/or lower viral load or viral RNA. In a preferred embodiment, the improvement consists in a 70% reduction, more preferably a 90% reduction in viral load or viral RNA after at least five days (for example by a real-time RT-PCR test).

According to a scale proposed by the World health Organization (WHO) the patients tested positive for SARS-CoV-2, can be grouped into the following 8 categories:
1. Not hospitalized, able to resume normal activities
2. Not hospitalized, but not able to resume normal activities
3. Hospitalization, not requiring supplemental oxygen
4. Hospitalization, requiring supplemental oxygen by mask or nasal prongs
5. Hospitalization, requiring non-invasive ventilation or high flow oxygen devices
6. Hospitalization, requiring intubation and mechanical ventilation
7. Hospitalization, requiring ventilation + additional organ support as pressors, Renal Replacement Therapies or Extracorporal Membrane Oxygenation (ECMO)
8. Death.

According to the present invention it has been found that the method of the second aspect or the oral solid unit dosage form of the third aspect is preferably administered to patients grouped in categories 1 to 5, more preferably categories 3 to 5, especially to patients of categories 3 to 5 within the first to seventh day, more preferably within the first to third day after being tested positive for SARS-CoV-2, because it was found that when treating these patients, the viral load could be reduced in a highly significant way.

In a further embodiment of said oral solid unit dosage form for use in the treatment of subjects suffering from a coronavirus-associated disease, preferably COVID-19, said oral solid unit dosage form is administered together (simultaneously or in any appropriate sequence) with one or more of the following additional drugs:
- lopinavir/ritonavir;
- favipiravir;
- remdesivir;
- hydroxychloroquine;
- chloroquine;
- methylprednisolone;
- hydrocortisone;
- budesonide;
- dexamethasone;
- prednisone;
- prednisolone;
- triamcinolone;
- betamethasone;
- mometasone;
- clobetasol;
- bicalutamide;
- flutamide;
- cyproteronacetate;
- darolutamide;
- enzalutamide;
- abiraterone;
- azithromycin;
- doxycycline;
- albendazole;
- duvelisib;
- infliximab;
- adalimumab;
- certolizumab;
- golimumab;
- etanercept;
- thalidomide;
- lenalidomide;
- pomalidomide;
- anakinra;
- rilonacept;
- canakinumab;
- tocilizumab;
- siltuximab;
- aifrolumab;
- anti-SARS-CoV-2 convalescent plasma;
- an autologous stem cell therapy; and
- a homologous stem cell therapy.

This invention will be better understood by reference to the non-limiting examples that follow, but those skilled in the art will readily appreciate that the examples are only illustrative of the invention and the claims which follow thereafter.

Unless otherwise indicated, all percentages of the ingredients in the present application are percentages by weight (w/w).

### EXAMPLES

### EXAMPLE 1

The process of one exemplary embodiment of the present invention is described below:

| | **mg/tablet** | **Percent (%)** |
|---|---|---|
| Active pharmaceutical ingredient | | |
| Ivermectin | 9.00 | 5.00 |

| Excipient | | |
|---|---|---|
| Microcrystalline cellulose | 142.00 | 78.96 |
| Pregelatinized corn starch | 28.00 | 15.00 |
| Citric acid | 0.75 | 0.50 |
| Butylated hydroxyanisole | 0.02 | 0.04 |
| Magnesium stearate | 0.23 | 0.5 |
| Total | 180.00 | 100.00 |

All the starting materials (ivermectin, microcrystalline cellulose, pregelatinized corn starch, citric acid, butylated hydroxanisole, are passed through a 40 mesh screen. Using geometric dilution principles, one third of the total microcrystalline cellulose is charged into a V-blender followed by the ivermectin and pregelatinized starch. The materials are mixed for 5 minutes. Citric acid and butylated hydroxyanisole are added to the mixer followed by another third of the total microcrystalline cellulose. The mixture is blended for 5 minutes. The remaining amount of microcrystalline cellulose is added to the blender and blended for 10 minutes. The materials are discharged from the blender and the blend is milled using a comminution mill (Fitzpatrick J- type) with hammers forward, at medium speed, using a 25 mesh screen. The milled blend is charged back into the V-blender followed by magnesium stearate. The mixture is blended for 5 minutes. The final blend is discharged from the blender and charged into the hopper of a tablet press. Tablets are compressed using a flat face, beveled edge tooling to a hardness of 50 N.

Content uniformity results are shown in the table below. All values are well within acceptable limits.

Dissolution results are shown in the table below:
Batch No.: IVFI-0401-9MG-001
Time Point: T=0 (release)

### Dissolutions conditions:

- Apparatus: USP (II) Paddles.
- Volume: 900 ml.
- Media: Buffer phosphate 0.01 M pH 7.0 + 0.5% SLS
- Speed (rpm): 50 rpm
- Time (min.): 5, 10, 15, 20, 30, 45

| Time (min) | V1 | V2 | V3 | V4 | V5 | V6 | AVERAGE (V1-V6) | DS (V1-V6) | RSD% (V1-V6) | MIN (V1-V6) | MAX (V1-V6) |
|---|---|---|---|---|---|---|---|---|---|---|---|
| 0 | 0,0 | 0,0 | 0,0 | 0,0 | 0,0 | 0,0 | 0 | 0 | 0 | 0 | 0 |
| 5 | 67,0 | 70,3 | 65,4 | 61,1 | 68,9 | 66,7 | 67 | 3 | 5 | 61 | 70 |
| 10 | 82,1 | 82,8 | 80,5 | 74,0 | 84,9 | 78,1 | 80 | 4 | 5 | 74 | 85 |
| 15 | 88,0 | 87,0 | 85,7 | 79,0 | 90,2 | 83,3 | 86 | 4 | 5 | 79 | 90 |
| 20 | 90,4 | 89,5 | 87,3 | 83,8 | 92,3 | 85,8 | 88 | 3 | 4 | 84 | 92 |
| 30 | 92,4 | 92,1 | 89,7 | 87,9 | 94,3 | 89,0 | 91 | 2 | 3 | 88 | 94 |
| 45 | 93,5 | 93,1 | 91,2 | 89,2 | 95,1 | 91,6 | 92 | 2 | 2 | 89 | 95 |

### EXAMPLE 2

*In vivo* experiment:
Ivermectin tablets as produced in Example 1 were administered for seven days in a daily dose of 600 µg/kg once daily to 9 patients (IVM) which have been tested to be positive for SARS-CoV-2, whereas 7 patients (control) which have been tested to be positive for SARS-CoV-2, received placebo tablets during said 7 days period.

The viral load was measured by a real-time (RT) PCR test on treatment days 1, 3, 5 and 7.

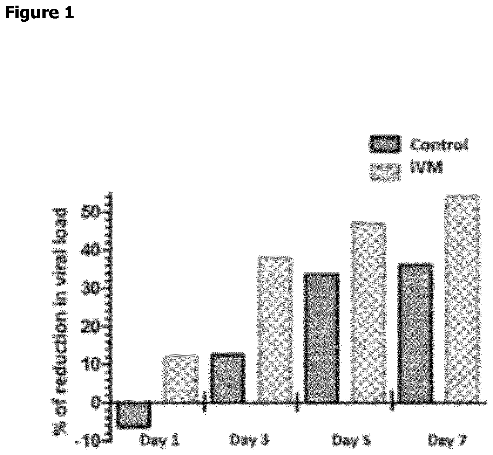

As can be seen from Figure 1 below, the 9 patients who received ivermectin showed a greater reduction of the viral load at each timepoint. At Day 7 the patients treated with ivermectin showed a significant reduction of more than 50% in viral load.

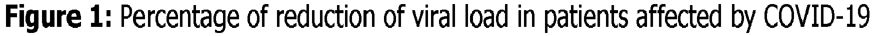

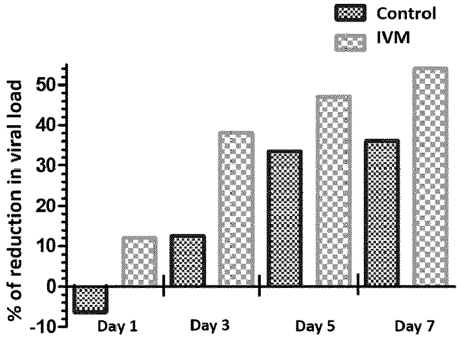

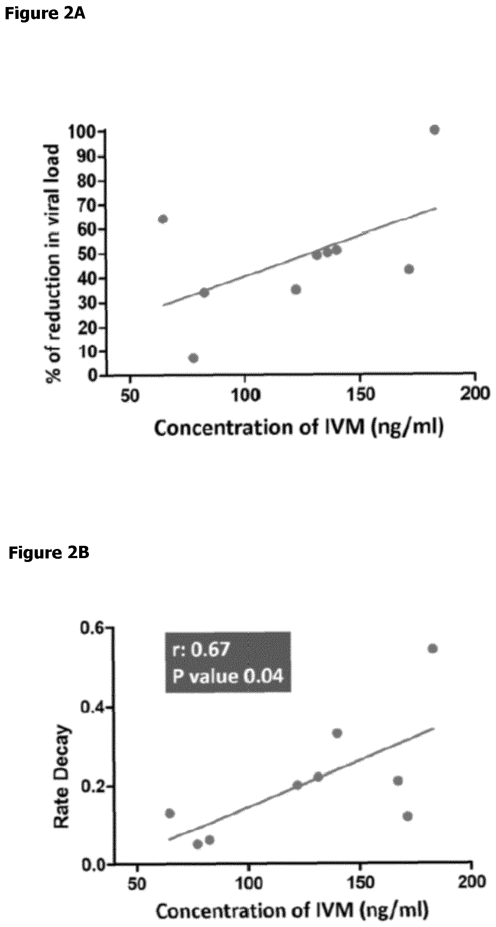

Furthermore, Figures 2A and 2B demonstrate that a higher ivermectin concentration in the blood after an oral administration of the ivermectin tablets apparently results in a significant viral load reduction (100% reduction for the patient with 183 ng/ml ivermectin (IVM) concentration in the blood).

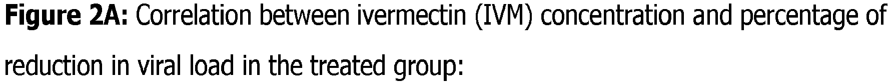

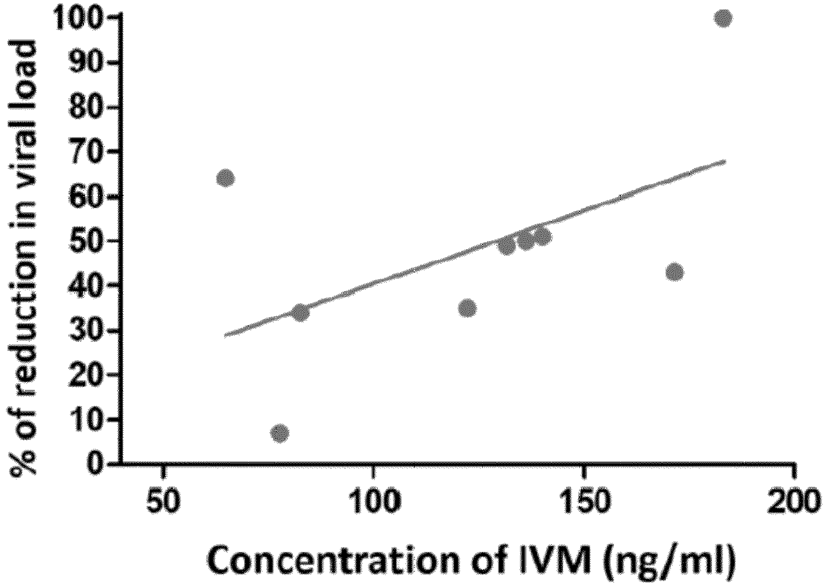

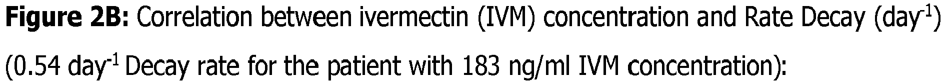

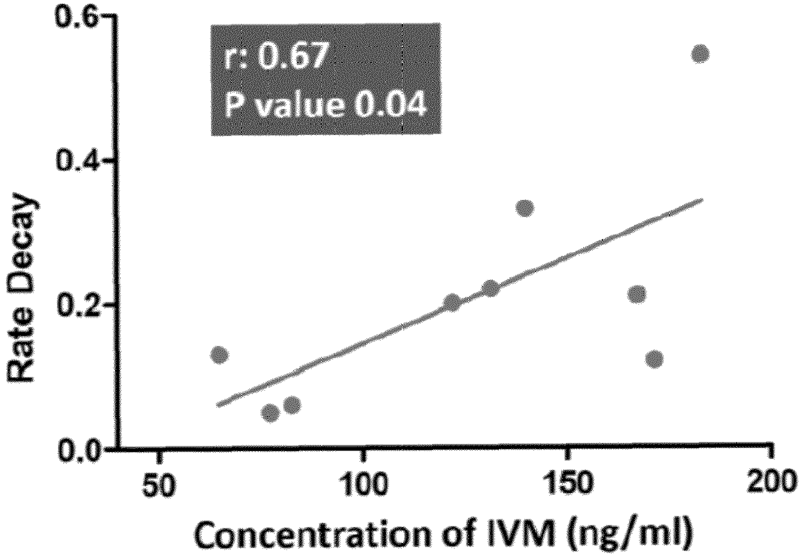

## Claims

1. An oral solid unit dosage form comprising an amount of 3 to 25 mg ivermectin or a pharmaceutically acceptable salt or solvate thereof in at least one pharmaceutically acceptable excipient.

2. The oral solid unit dosage form as claimed in claim 1, wherein the unit dosage form is an immediate-release unit dosage form, wherein more than 75% of the ivermectin contained in the unit dosage form are dissolved in the dissolution medium within 45 minutes under agitation when using an USP device 2 (paddle) at 50 rpm in 0.01M phosphate buffer with 0.5% of sodium dodecyl sulfate, pH 7 (USP43-NF38).

3. The oral solid unit dosage form as claimed in claim 1 or 2, wherein the ivermectin or a pharmaceutically acceptable salt or solvate thereof present in the unit dosage form, is crystalline ivermectin.

4. The oral solid unit dosage form as claimed in any one of claims 1 to 3, wherein the crystalline ivermectin has a particle size distribution D(v, 0.5) of 10 to 25 µm.

5. The oral solid unit dosage form as claimed in any one of claims 1 to 4, wherein the at least one pharmaceutically acceptable excipient is selected from the group consisting of binders/fillers; disintegrants; glidants; lubricants; colorants; flavoring agents; preservatives; chelating agents and pH modifiers.

6. The oral solid unit dosage form as claimed in any one of claims 1 to 5, wherein the oral solid unit dosage form is coated with a coating agent selected from the group consisting of ethylcellulose, methyl hydroxyethyl cellulose, povidone, gelatin, hydroxypropyl cellulose, hypromellose, cellulose acetate phthalate, an acrylate polymer or hydroxymethyl propyl cellulose.

7. A method of treating subjects suffering from a coronavirus-associated disease, preferably COVID-19 by administering an oral solid unit dosage form comprising an amount of 3 to 25 mg ivermectin in at least one pharmaceutically acceptable carrier.

8. The method of treating subjects suffering from a coronavirus-associated disease according to claim 7, by administering an amount of 480 to 800 µg/kg/day ivermectin or a pharmaceutically acceptable salt or solvate thereof over a period of one to seven days.

9. An oral solid unit dosage form comprising ivermectin or a pharmaceutically acceptable salt or solvate thereof in at least one pharmaceutically acceptable carrier for use in the treatment of subjects suffering from a coronavirus-associated disease, preferably from COVID-19.

10. The oral solid unit dosage form for use according to claim 9, wherein said ivermectin is present in an amount of from 3 to 25 mg, preferably 5 to 21 mg ivermectin or a pharmaceutically acceptable salt or solvate thereof, more preferably 7 to 20 mg, more preferably 8 to 19 mg, even more preferably 9 to 18 mg.

11. The oral solid unit dosage form for use according to claim 9 or 10, wherein the oral solid unit dosage form is an immediate-release unit dosage form, wherein more than 75% of the ivermectin contained in the unit dosage form are dissolved in the dissolution medium within 45 minutes under agitation when using an USP device 2 (paddle) at 50 rpm in 0.01M phosphate buffer with 0.5% of sodium dodecyl sulfate, pH 7 (USP43-NF38).

12. The oral solid unit dosage form for use according to any one of claims 9 to 11, wherein the unit dosage form is an immediate-release oral dosage form comprising an amount of 8 to 20 mg of ivermectin.

13. The oral solid unit dosage form for use according to any one of claims 9 to 12, wherein said unit dosage form is administered to a patient in an amount of 500 to 800 µg/kg/day ivermectin or a pharmaceutically acceptable salt or solvate thereof over a period of one to seven days.

14. The oral solid unit dosage form for use according to claim 13, wherein said unit dosage form is administered to a patient in an amount of 480 to 800 µg/kg/day ivermectin for two to five days.

15. The oral solid unit dosage form for use according to any one of claims 9 to 14, wherein said unit dosage form is administered in combination with one or more of the following additional drugs or therapies:
- lopinavir/ritonavir;
- favipiravir;
- remdesivir;
- hydroxychloroquine;
- chloroquine;
- methylprednisolone;
- hydrocortisone;
- budesonide;
- dexamethasone;
- prednisone;
- prednisolone;
- triamcinolone;
- betamethasone;
- mometasone;
- clobetasol;
- bicalutamide;
- flutamide;
- cyproteronacetate;
- darolutamide;
- enzalutamide;
- abiraterone;
- azithromycin;
- doxycycline;
- albendazole;
- duvelisib;
- infliximab;
- adalimumab;
- certolizumab;
- golimumab;
- etanercept;
- thalidomide;
- lenalidomide;
- pomalidomide;
- anakinra;
- rilonacept;
- canakinumab;
- tocilizumab;
- siltuximab;
- aifrolumab;
- anti-SARS-CoV-2 convalescent plasma;
- an autologous stem cell therapy; and
- a homologous stem cell therapy.
